# EUROPEAN PATENT SPECIFICATION

(11) **EP 4 409 297 B1**
(45) Date of publication and mention of the grant of the patent: **29.01.2025**
(21) Application number: 22800159.0
(22) Date of filing: 03.10.2022
(51) Int. Cl.: G01N 33/68

(54) **NOVEL METHOD FOR DETERMINING A CONCENTRATION OF N-TERMINAL PRO-HORMONE BNP (NT-PROBNP) IN A SAMPLE**
NEUES VERFAHREN ZUR BESTIMMUNG EINER KONZENTRATION VON N-TERMINALEM PRO-HORMON-BNP (NT-PROBNP) IN EINER PROBE
NOUVEAU PROCÉDÉ DE DÉTERMINATION D'UNE CONCENTRATION DE PRO-HORMONE BNP N-TERMINALE (NT-PROBNP) DANS UN ÉCHANTILLON

(30) Priority: 01.10.2021 US 202117492372
(43) Date of publication of application: 07.08.2024
(73) Proprietor: Gentian AS, 1509 Moss (NO)
(72) Inventor: SUNDREHAGEN, Erling, 1509 Moss (NO); GUDIM, Ingvild, 1509 Moss (NO); WEIKUM, Julia, 1509 Moss (NO); DAVID, Arnaud, 1435 Ås (NO); KNÜTTEL, Torsten, 1424 Ski (NO)
(74) Representative: Kransell & Wennborg KB
(86) International application number: PCT/EP2022/077433
(87) International publication number: WO 2023/052642

(56) References cited:
- WO-A1-2012/166918
- WO-A1-2021/028520
- WO-A2-2007/047614
- WO-A2-2012/058313
- US-A1- 2012 034 710

## Description

### Technical field

This disclosure relates to the field of diagnostic assays, and in particular to assays for determination of a concentration of N-terminal pro-hormone BNP (NT-proBNP) in a sample, including methods and reagents for performing such assay, such as antibodies, immunoparticles, and calibrators. The disclosure presents a new and more accurate method, particularly useful for assigning the value to calibrators for use in diagnostic assays for the determination of a concentration of N-terminal pro-hormone BNP (NT-proBNP) in a sample.

### Background

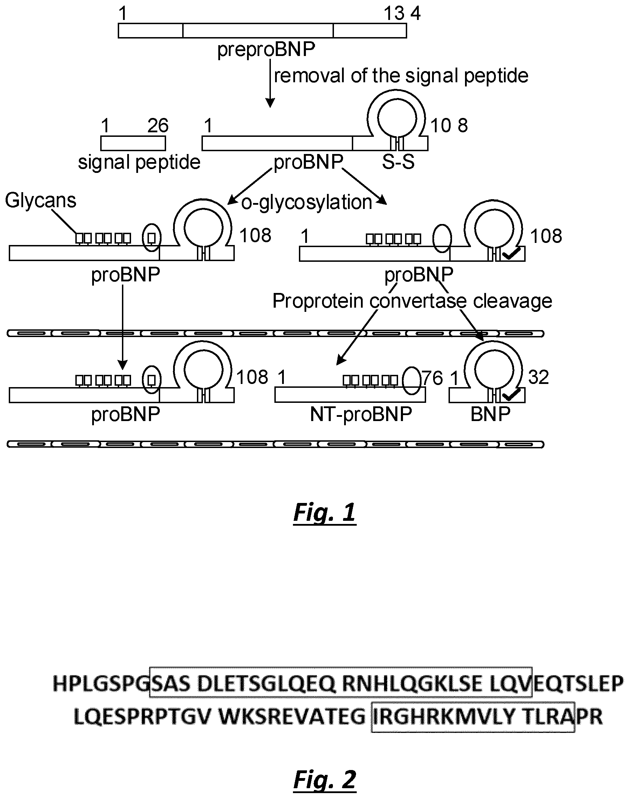

Brain natriuretic peptide, also known as B-type natriuretic peptide, abbreviated BNP, is a hormone secreted by cardiomyocytes in the heart ventricles in response to stretching caused by increased ventricular blood volume, cardiac wall stress. NT-proBNP is produced by cleaving the proBNP into BNP and the NT-proBNP. See Figure 1.

Measuring BNP and NT-proBNP is useful in the diagnosis and monitoring of heart failure. BNP and NT-proBNP cannot be compared directly, as BNP is the biological active metabolite and has a half-life (t_{1/2}) of approx. 20 min and does not degrade in the kidneys. It is only stable for a very short time *in vitro.*

NT-proBNP is however more stable, it is biologically inactive and filtered by the kidneys. Its half-life is about 1 to 2 hours and the concentration in the blood is higher than that of BNP. Compared to BNP, the half-life is highly dependent of kidney function. *In vitro,* NT-proBNP is more stable than BNP, and can be stored for at least three days at 2 - 8 °C. Consequently, proBNP and NT-proBNP are used as gold standard clinical markers of myocardial dysfunction such as cardiac hypertrophy and left ventricle heart failure.

The recommended reference range limits to exclude heart failure in patients with non-acute symptoms are currently 35 ng/L and 125 ng/L for BNP and NT-proBNP respectively. The recommended reference range limits to exclude heart failure in patients with dyspnoea or increasing symptoms are higher, 100 ng/L and 300 ng/L for BNP and NT-proBNP respectively. The reference range is also wide, in particular for acute heart failure, and in particular for older patients, see Table 1:

**Table 1. Age-stratified reference range limits for the diagnosis of acute heart failure**

| **Age** | **Reference range limit** | **Sensitivity, %** |
|---|---|---|
| < 50 years | < 450 pg/mL | 97 |
| 50 - 75 years | < 900 pg/mL | 90 |
| > 75 years | < 1800 pg/mL | 85 |

(Januzzi et al., NT-proBNP testing for diagnosis and short-term prognosis in acute destabilized heart failure: an international pooled analysis of 1256 patients. Eur Heart J 2006; 27: 330-37)

NT-proBNP is a peptide chain of 76 amino acids with a mass of 8458 Da in its non-glycosylated form. The amino acid sequence is shown in Fig. 2. Before 2006, the glycosylation of proBNP was unknown. The first evidence of proBNP glycosylation was published in 2006 (Schellenberger, U. et al., The precursor to B-type natriuretic peptide is an O-linked glycoprotein, Arch Biochem Biophys. 2006 Jul 15;451(2):160-6. Epub 2006 Apr 19). The presence of glycosylation and the observations of higher molecular weight isoforms of BNP have caused much confusion about the relevant forms of NT-ProBNP in the blood stream.

In 2015, it was shown that higher NT-proBNP concentrations were measured using the proBNP II assay on an Elecsys^{®} platform (Roche Diagnostics) when the samples were pre-treated with a deglycosylation enzyme (Røsjø et al., Influence of glycosylation on diagnostic and prognostic accuracy of N-terminal pro-B-type natriuretic peptide in acute dyspnoea: data from the Akershus Cardiac Examination 2 Study, Clin Chem, 2015 Aug;61(8):1087-97. doi: 10.1373/clinchem.2015.239673. Epub 2015 Jun 8.).

The exact glycosylation sites of endogenous proBNP and NT-proBNP were only recently characterized (Halfinger, B. et al., Unravelling the Molecular Complexity of O-Glycosylated Endogenous (N-Terminal) pro-B-Type Natriuretic Peptide Forms in Blood Plasma of Patients with Severe Heart Failure. Clinical Chemistry. 2017, Vol. 63, 1).

Sandwich immunoassays for the detection of NT-proBNP are commercially available, and disclosed for example in EP 1151304 (Roche Diagnostics GmbH) which relates to a sandwich assay using two antibodies which recognize different epitopes of the native NT-proBNP and at the same time can bind to said native NT-proBNP, wherein at least two antibodies bind in the region of amino acid 10-50 of N-terminal proBNP and the antibodies are obtained by immunizing a suitable organism with recombinant NT-proBNP.

EP 1625163, EP 1625164 and EP 2256132 (Roche Diagnostics GmbH) define antibodies specific to epitopes on the proBNP molecule defined by amino acids no. 38-43, 42-46, 39-66, 40-47, 41-48 and 42-49.

US 9,034,591 and US 9,145,459 (HyTest Ltd.) both relate to an immunoassay kit for detecting BNP, proBNP or a fragment thereof, in a sample, comprising: a first antibody or binding fragment thereof, specific for a region of a ring structure of BNP and proBNP; wherein the first antibody or binding fragment thereof, binds to BNP, proBNP or to a fragment thereof comprising the region thereof so as to form a first order immune complex; and a second antibody or binding fragment thereof, not recognizing free BNP, free proBNP or a free fragment thereof, or free first antibody, or which recognizes them with 10-fold or less affinity than it recognizes the first order immune complex, wherein, the second antibody or binding fragment thereof, is Ab-BNP2 or Ab-BNP4.

EP2084544B1 (HyTest Ltd.) relates to stable standards for BNP immunoassays and defines the use of a peptide selected from the group consisting of an isolated or recombinant or synthetic proBNP consisting of a specific amino acid sequence or a sequence that differs by five or fewer amino acid substitutions, insertions or deletions as a standard or calibrator in a method for detecting BNP immunoreactivity in a sample.

EP2251356B1 (Nexus DX Inc.) relates to a polyclonal-monoclonal ELISA assay for detecting N-terminal proBNP using isolated polyclonal antibodies specific for NT-proBNP within a certain range of amino acid residues, and isolated monoclonal antibodies specific for NT-proBNP within another, specific range of amino acid residues.

Comparison of existing clinical assays for the determination of NT-proBNP shows that the existing assays are still not standardised, and that the same samples yield different values depending on which assay is used. See for example Collin-Chavagnac et al., Head-to-head comparison of 10 natriuretic peptide assays, Clinical Chemistry and Laboratory Medicine, April 2015, Doi: 10.1515/cclm-2014-0592.

Nevertheless, NT-proBNP has been called "the gold standard biomarker in heart failure" (McKie and Burnett, J Am Coll Cardiol, 2016 Dec 6;68(22):2437-2439). The current deviation between values obtained using different assays is however not satisfactory, and there is a need for an independent reference method for determining the concentration of NT-proBNP.

US 20080312152 (Pollit et al.) discusses the therapeutic use of proBNP in pharmaceutical compositions and determines high concentrations of recombinant proBNP in a regular buffer. Pollit et al. do not show, teach or suggest how physiological amounts of NT-proBNP could be determined in plasma samples.

US 20050148024 (Buechler) discloses a method focused on degradation of proBNP and determining the presence or amount of one or more natriuretic peptides, or their fragments, in a sample, the method designed to distinguish between various natriuretic peptides.

In the article "Standardization of BNP and NT-proBNP Immunoassays in Light of the Diverse and Complex Nature of Circulating BNP-Related Peptides", the authors Alexander G. Semenov and Evgeniya E. Feygina (Semenov, 2018, Advances in Clinical Chemistry, Volume 85, pages 1-30) note that there is no agreement on what kind of BNP or NT-proBNP standard should be used for calibration, and a certified reference material as well as reference measurement procedures are lacking.

WO2012/058313 discloses a method for determining a concentration of human N-terminal pro-hormone BNP (NT-proBNP) in a sample but it does not use high affinity epitopes for a non-glycosylated epitope and there is no step of trypsinization.

### Summary

The present inventors have addressed the above problem, and others, by making available a robust and reliable method suitable for serving either as a reference method for calibrating and ensuring the accuracy of existing methods, or as a novel method in clinical chemistry for accurately determining the concentration of NT-proBNP.

The broadest aspect of the claimed invention is defined in independent claim 1. Preferred embodiments are defined in claims 2-12.

The method defined in the claims qualifies as a higher order method or reference method, meaning that it is a high-accuracy method that can be used as a diagnostic method as such, but also for assigning values to calibration materials. Generally, the term reference method infers a high-quality method whose results are known to be correct. Any differences between a test method and a reference method are assigned to the test method, i.e., the errors are attributed to the test method because the correctness of the reference method is well documented.

According to a first aspect the present disclosure makes available a method for determining a concentration of human N-terminal pro-hormone BNP (NT-proBNP) in a sample, wherein said method comprises
- extracting and isolating NT-proBNP from the sample using an affinity purification method,
- adding a least one labelled peptide standard to said isolated NT-proBNP,
- subjecting said isolated NT-proBNP and peptide standard to enzymatic digestion, producing a mixture of peptides, and
- detecting at least one peptide in the resulting mixture, using liquid chromatography-mass spectrometry analysis with subsequent mass spectrometric detection, wherein said at least one peptide to be detected is chosen from NHLQG(K) (SEQ ID NO. 11), EVATEGI(R) (SEQ ID NO. 12), and MVLYTL(R) (SEQ ID NO. 13).

In the above sequences, (K/R) denotes trypsin cleavage sites.

According to an embodiment, freely combinable with any of the above embodiments, said at least one labelled peptide standard is full-length recombinant NT-proBNP.

According to an embodiment, freely combinable with any of the above embodiments, said at least one peptide standard is chosen from NHLQG(K) (SEQ ID NO. 11), EVATEGI(R) (SEQ ID NO. 12), and MVLYTL(R) (SEQ ID NO. 13).

According to a specific embodiment, freely combinable with any of the above embodiments, said at least one peptide standard is EVATEGI(R) (SEQ ID NO. 12), preferably a deuterium labelled EVATEGI(R) (SEQ ID NO. 12).

According to an embodiment, freely combinable with any of the above embodiments, said affinity purification method makes use of affinity beads carrying at least one antibody against said NT-proBNP, and wherein said at least one antibody exhibits high affinity for a non-glycosylated epitope on the NTproBNP molecule. Preferably said affinity beads are magnetic affinity beads.

According to an embodiment, freely combinable with any of the above embodiments, said at least one antibody binds specifically to a non-glycosylated epitope on the NT-proBNP within an amino acid sequence chosen from SEQ ID NO. 2 and SEQ ID NO. 3.

According to an embodiment of the first aspect, freely combinable with any of the above embodiments, said enzymatic digestion is a trypsinization. Preferably said trypsinization is performed while the labelled standard and native NT-proBNP are associated with the beads.

According to an embodiment, freely combinable with any of the above embodiments, quantitation is performed based on calibration against enzymatically digested recombinant NT-proBNP.

According to an embodiment, freely combinable with any of the above embodiments, the method is calibrated using trypsinated standard NT-proBNP at relevant concentrations, and wherein all standards are spiked with an internal standard. Preferably the internal standard is labelled recombinant full-length NT-proBNP.

Another aspect relates to a method for assigning a value to calibration materials, testing and/or quality control of assays for determining a concentration of human N-terminal pro-hormone BNP (NT-proBNP) in a sample, wherein said method comprises the steps outlined in the first aspect and embodiments thereof.

A third aspect of the present disclosure relates to a method for use in the diagnosis and monitoring of heart failure, wherein said method comprises the steps outlined in the first aspect and embodiments thereof.

Further aspects and embodiments and their advantages will emerge from the following detailed description, examples, and the attached claims and drawings.

### Brief description of the drawings

The invention is now described, by way of example, with reference to the accompanying drawings, in which:
Figure 1 shows the scheme of proBNP processing (Source: TechNotes | Human ProBNP, BNP and NT-proBNP, HyTest Ltd., January 2019,https://shop.hytest.fi/spree/products/2932/Human_proBNP_BNP_and_NT-proBNP_TechNotes.pdf?1560757320, downloaded 07.08.2019).
   ProBNP is formed following the translation and cleavage of the signal peptide of pre-proBNP molecule. It is then glycosylated at several sites. Two pools of proBNP that are different in the status of T71 glycosylation are formed: non-glycosylated at T71 and molecules glycosylated at this site. Glycosylation suppresses the subsequent processing of proBNP. Only proBNP that is not glycosylated at T71 can be effectively processed into BNP and NT-proBNP. Non-processed proBNP, NT-proBNP and BNP are released into the blood.
Figure 2 presents the amino acid sequence of NT-proBNP as amino acids 27-102 of NCBI Reference Sequence NP_002512, with two peptides previously identified by the present inventors indicated: amino acid positions 8 - 33 (Peptide 1) and 61 - 74 (Peptide 2).
Figure 3 is a graph showing the correlation between the concentration of NT-proBNP plasma samples measured using the herein presented method and values assigned to the samples using a commercial method belonging to the prior art (the Roche Elecsys^{®} NT-proBNP assay) in the lower concentration range (0 - 2000 ng/L).
Figure 4 is a graph showing the correlation between the concentration of NT-proBNP plasma samples measured using the herein presented method and values assigned to the samples using a commercial method belonging to the prior art (the Roche Elecsys^{®} NT-proBNP assay) over a wider concentration range (0 to 25000 ng/L).
Figure 5 is a graph showing the correlation between the concentration of NT-proBNP plasma samples measured using the herein presented method and values assigned to the samples using another commercial method (the Siemens ADVIA Centaur^{®} Immunoassay System).
Figure 6 is a graph showing the correlation between the concentration of NT-proBNP of serum samples measured using the herein presented method and values assigned to the samples using another commercial method (the Siemens IMMULITE Immunoassay System).

### Detailed description

Before the present invention is described in more detail, it is to be understood that the terminology employed herein is used for the purpose of describing particular embodiments only and is not intended to be limiting, since the scope of the present invention will be limited only by the appended claims and equivalents thereof.

It must be noted that, as used in this specification and the appended claims, the singular forms "a," "an," and "the" include plural referents unless the context clearly dictates otherwise.

The term "sample" as in "a sample of whole blood" refers to a sample taken from a human or animal body, and which sample will not be returned to said human or animal body. There are standardized methods for obtaining and handling a blood sample taken from a human or animal body, involving the use of needles, syringes, micro cuvettes etc. These methods are well-known to persons skilled in the art. The currently most preferred type of sample is a lithium heparin treated sample of whole blood. There are several blood collection tubes containing spray coated lithium heparin readily available from various commercial supplies, e.g. the BD Vacutainer^{®} available from BD, Oakville, Ontario, Canada.

The term "immunogen" in this disclosure refers to substances used to produce antibodies and includes conjugated and unconjugated forms.

The term "specifically binds to NT-proBNP within the amino acid sequence of ..." is understood to mean that only amino acids within the specified amino acid stretch of NT-proBNP are responsible for being specifically recognized by and/or binding to the antibody. In other words, an antibody as described herein specifically recognizes and/or binds to an epitope within a given amino acid stretch of NT-proBNP.

The term "specifically recognizes an epitope" or "specifically binds an epitope" is understood to mean that only the amino acids of the epitope are responsible for the binding to the antibody. Accordingly, the term "epitope" denotes an antigenic determinant, namely a specific part or parts of an antigen such as NT-proBNP to which an antibody binds (such as an epitope comprising part or all amino acids found in the amino acid sequences corresponding to SEQ ID NO: 2 or 3). None of the other amino acids of an antigen such as NT-pro-BNP are supposed to be involved in the binding of the antibody. Lack of cross-reactivity of an antibody to other molecules means that the antibody has high epitope specificity.

Further, "binding with substantially the same affinity" is understood to mean that an antibody binds for example to two or more different peptides with binding affinities in the same order of magnitude. The antibody or antibodies as described herein bind to a peptide, e.g. an 8-mer peptide in the context of an epitope mapping assay, or to a protein, with an affinity of at least about 50%, of at least about 60%, of at least about 70%, of at least about 80%, of at least about 90%, of at least about 95%, of at least about 97%, of at least about 98%, or of at least about 99% of the affinity of the same antibody or antibodies binding to another peptide or protein.

As used herein, the term "at least one antibody or antibodies" refers to polyclonal antibodies, a mixture of monoclonal antibodies of any isotype (IgA, IgG, IgD, IgM, IgY) or an antigen-binding fragment thereof, including but not limited to F(ab), F(ab'), F(ab')2, Fv fragments, single chain antibodies such as scFv, chimeric antibodies, humanized antibodies and a Fab expression library.

The terms "binding fragment of an antibody" or "antibody fragment" refer to a fragment of a full-length antibody which results e.g., from deletion of N-terminal or C-terminal amino acids of the full-length antibody and which maintains the capacity to bind the cognate antigen with about the same specificity and/or K_{D} as the full-length antibody.

Assays to determine binding specificity and K_{D} of an antibody are well known and routinely practiced in the art. For a comprehensive discussion of such assays, see Harlow et al. (Eds.), Antibodies: A Laboratory Manual; Cold Spring Harbor Laboratory; Cold Spring Harbor, NY (1988), Chapter 6.

One approach to measure specificity is by testing an antibody for binding to its cognate antigen and other non-related antigens using Western Blotting or ELISA. Another approach to measure affinity of binding of an antibody to its antigen is by surface plasmon resonance (SPR) using for example the Biacore^{™} assay platform and software (GE Healthcare).

Blood tests, involving laboratory examination of a sample of blood to obtain information about its physical and chemical properties, today form the foundation of medical diagnosis. Hundreds of haematological tests and procedures have been developed over the years. While the tests were originally performed manually, one by one, the field of clinical chemistry has become highly automated. Today multiple tests can be carried out simultaneously on one single sample of blood using automated clinical chemistry analysers, instruments frequently referred to as autoanalyzers.

While the presently disclosed assay method can be performed manually, the assay is preferably performed on an autoanalyzer, as this allows high throughput, reduces error, and minimizes staffing requirements. A closed, automated sample handling also minimizes the risk that staff is exposed to possible blood borne pathogens.

When taking a blood sample, blood is usually drawn from a vein in the arm and collected in standardized sterile blood collection tubes. Different tubes are available, depending on which component or components that is to be analysed. A blood collection tube can be empty, or prefilled with a buffer or more frequently, with an anticoagulant. Examples of anticoagulants include sodium citrate, lithium-heparin, and EDTA. Different combinations are also possible, there are for example tubes containing sodium heparin and EDTA. The Vacutainer^{™} (BD, Becton, Dickinson and Company) is one example of blood collection tubes, available in different sizes and pre-filled with various reagents.

A distinction is made between plasma samples and serum samples. When a plasma sample is desired, blood is collected in a tube containing an anticoagulant and the tube is turned upside down about 5 to 10 times directly after filling, in order to ensure mixing. The tubes are then stored in an upright position in room temperature to avoid haemolysis. EDTA and lithium heparin containing tubes can generally be stored up to 4 hours in room temperature. Alternatively, the tubes can be deep-frozen and stored for later analysis.

For obtaining a serum sample, blood is collected in an empty sample tube or a tube containing a coagulation activator. After filling, the tubes are turned upside down about 5 to 10 times directly after filling, in order to ensure mixing. The tubes are then stored in an upright position in room temperature to avoid haemolysis. In most cases, serum tubes can be stored in room temperature and without centrifuging for up to 4 hours. Upon arrival in the laboratory, the tubes are centrifuged, for example centrifuged for 10 minutes at 2000 x g. Following centrifugation, a layer of blood cells can be seen at the bottom of the tube, and the fluid above is called serum. The serum should be separated and stored cold without any additives until transport or analysis.

The inventors note that immunoassay systems require calibration protocols that are normally more sophisticated than many analytical techniques in routine clinical use. Calibrators used in such assays may differ significantly from the analyte in clinical specimens. Differences in the properties of calibrators, or reference materials, from those of clinical specimens may include the species origin of the calibrator for an analyte; integrity of the molecular species; matrix of the calibration solution, and addition of preservative agents.

Brain natriuretic peptide (BNP) and the N-terminal fragment of the BNP precursor (NT-proBNP) are widely used biomarkers for heart failure (HF). Since the discovery of BNP in 1988, much effort has been allocated to the precise detection of BNP and NT-proBNP levels for reliable HF diagnostics. As a result, measurements of these biomarkers are globally accepted and used in clinical practice for the diagnosis of acute and chronic HF, risk stratification, and monitoring response to therapy. Several immunoassays specific for BNP and NT-proBNP are currently commercially available.

The multiple circulating BNP fragments, along with proBNP and NT-proBNP, collectively form the B-type natriuretic peptide family. Immunoassays for BNP utilize a variety of antibodies, both monoclonal and polyclonal, and diverse calibrator materials. Therefore, there are substantial differences in patient test values between BNP methods, even for assays that use identical antibody configurations on different analytical platforms.

Previous studies have demonstrated that proBNP and NT-proBNP are glycosylated to varying degrees that can interfere with commercial immunoassays used to quantify NPs in clinical practice (e.g., Saenger *et al.,* 2017). The extent to which these immunoassays exhibit cross-reactivity to B-type natriuretic peptides as well as with their glycosylated and non-glycosylated forms is an important question to elucidate, as this may affect the clinical performance of the assays with implications for patient care. Furthermore, there are currently no formal efforts to standardize or maintain harmonization of BNP or NT-proBNP assays, primarily owing to the known differences in antibodies used and lack of a primary reference standard material.

Recent comparative studies however indicate that there are marked differences between different BNP and NT-proBNP assays and platforms, and the results of measurements are not sufficiently comparable. One such comparison is presented in Example 1 (Collin-Chavagnac et al., 2015). The present inventors realized the significance and consequences of this, and the method outlined in the present description and claims offers an advantageous solution.

This lack of equivalence between the assays complicates the interpretation of the results and as a consequence, the reference range limits used for diagnostic decisions are in fact method dependent. As a consequence, diagnosis and decisions regarding therapy may vary, or even be arbitrary depending on which method was used for analysing the patient's samples. Presently, there also does not appear to be any agreement on what kind of BNP or NT-proBNP standard should be used for calibration, and certified reference material as well as reference measurement procedures are lacking.

According to a first aspect the present disclosure makes available a method for determining a concentration of human N-terminal pro-hormone BNP (NT-proBNP) in a sample, wherein said method comprises
- extracting and isolating NT-proBNP from the sample using an affinity purification method,
- adding a least one labelled peptide standard to said isolated NT-proBNP,
- subjecting said isolated NT-proBNP and peptide standard to enzymatic digestion, producing a mixture of peptides, and
- detecting at least one peptide in the resulting mixture, using liquid chromatography-mass spectrometry analysis with subsequent mass spectrometric detection, wherein said at least one peptide to be detected is chosen from NHLQG(K) (SEQ ID NO. 11), EVATEGI(R) (SEQ ID NO. 12), and MVLYTL(R) (SEQ ID NO. 13).

In the above sequences, (K/R) denotes trypsin cleavage sites.

According to an embodiment, freely combinable with any of the above embodiments, said at least one labelled peptide standard is full-length recombinant NT-proBNP.

According to an embodiment, freely combinable with any of the above embodiments, said at least one peptide standard is chosen from NHLQG(K) (SEQ ID NO. 11), EVATEGI(R) (SEQ ID NO. 12), and MVLYTL(R) (SEQ ID NO. 13).

According to a specific embodiment, freely combinable with any of the above embodiments, said at least one peptide standard is EVATEGI(R) (SEQ ID NO. 12), preferably a deuterium labelled EVATEGI(R) (SEQ ID NO. 12).

According to another embodiment, freely combinable with any of the above embodiments, one peptide standard is used quantitatively, to confirm the quantification of NT-proBNP in the sample, whereas another peptide standard is used qualitatively, to confirm that it actually is NT-proBNP that is detected and quantified in the assay.

According to an embodiment, freely combinable with any of the above embodiments, said affinity purification method makes use of affinity beads carrying at least one antibody against said NT-proBNP, and wherein said at least one antibody exhibits high affinity for a non-glycosylated epitope on the NTproBNP molecule. Preferably said affinity beads are magnetic affinity beads. Said at least one antibody is chosen from monoclonal and polyclonal antibodies exhibiting high affinity for a non-glycosylated epitope on the NTproBNP molecule.

According to an embodiment, freely combinable with any of the above embodiments, said at least one antibody binds specifically to a non-glycosylated epitope on the NT-proBNP within an amino acid sequence chosen from SEQ ID NO. 2 and SEQ ID NO. 3.

According to an embodiment of the first aspect, freely combinable with any of the above embodiments, said enzymatic digestion is a trypsinization. Preferably said trypsinization is performed while the labelled standard and native NT-proBNP are associated with the beads.

According to an embodiment, freely combinable with any of the above embodiments, quantitation is performed based on calibration against enzymatically digested recombinant NT-proBNP.

According to an embodiment, freely combinable with any of the above embodiments, the method is calibrated using trypsinated standard NT-proBNP at relevant concentrations, and wherein all standards are spiked with an internal standard. Preferably the internal standard is labelled recombinant full-length NT-proBNP.

In the past, determining the true concentration of NT-proBNP has been challenging, as the variations between results obtained with different assays has shown. The approach outlined above has several advantages, one being that variations in glycosylation no longer impact the results. Another advantage is that the herein disclosed method has a significantly improved sensitivity. The inventors have also proven that the method is robust and transferrable to different sites, where it has been performed by different operators and on different instruments. The claimed method can thus function as an important tool in research, in the calibration of existing assays, for the development of new assays, and as a diagnostic assay in itself.

A third aspect of the present disclosure relates to a method for use in the diagnosis and monitoring of heart failure, wherein said method comprises the steps outlined in the first aspect and embodiments thereof.

The claimed method thus presents an independent approach to quantifying NT-proBNP and offers a possibility to calibrate and standardize existing methods, and it will be useful in the production of standards, quality assurance and in the supervision of clinical laboratories. In other words, the invention offers an approach to level up existing assays.

Further, the claimed method exhibits a significantly improved sensitivity, making it possible to detect and accurately quantify a low-abundant protein such as NT-proBNP. The claimed method can also be used to generate data making it possible to recalculate and transfer results obtained with different methods, thus creating an uninterrupted medical history for a patient who has been subject to different analyses during the course of his/her illness, and in contact with various care providers.

Above all, the present method makes available a reliable method for detecting and quantifying an elusive biomarker.

Specific advantages have been presented in association with selected features and other advantages will be apparent to a person skilled in the art upon study of the following examples which illustrate the inventions.

### Examples

### Example 1. Prior art comparison of existing NT-proBNP assays

In the article "Head-to-head comparison of 10 natriuretic peptide assays" Delphine Collin-Chavagnac *et al.* report the results of their comparison of NT-proBNP and BNP levels in fresh samples from heart failure (HF) patients measured using 10 different immunoassays. NT-proBNP (CobasH232^{®}, Elecsys^{®}, Vidas^{®}, Vista^{®}, XPand^{®}, Vitros^{®}) and BNP (Triage^{®}, Access^{®}, CentaurXP^{®}, Architect^{®}) levels were measured in 39 heparin and 19 EDTA samples, respectively.

The Pearson correlation coefficient ranged between 0.929 (Triage^{®}-Centaur^{®}) and 0.994 (Access^{®}-Architect^{®}) for BNP assays and between 0.972 (Vidas^{®}-Cobas H232^{®}) and 0.999 (Vitros^{®}-Vidas^{®}) for NT-proBNP assays. Passing Bablok regression analyses showed a significant difference in the slopes [0.80 (Centaur^{®}-Triage^{®}) to 1.84 (Architect^{®}-Centaur^{®})] and intercepts [-55 ng/L (Architect^{®}-Centaur^{®}) to 48 ng/L (Access^{®}-Triage^{®})] for BNP assays, and a lower heterogeneity between NT-proBNP assays [0.83 (Vidas^{®}-Elecsys^{®}) to 1.20 (Vitros^{®}-Vidas^{®}) and -97 ng/L (XPand^{®}-CobasH232^{®}) to 51 ng/L (CobasH232^{®}-Elecsys^{®}) for slopes and intercepts, respectively].

The concordance correlation coefficient revealed a poor (pc<0.90) to moderate (pc=0.90-0.95) agreement in 4/6 pairs of BNP assays and an almost perfect (pc>0.99) agreement in 5/15 pairs of NT-proBNP assays. The acceptable difference limit reflecting the number of individual discrepant results between two assays, ranged between 15.1% (Access^{®}-CentaurXP^{®}) and 34.5% (Architect^{®}-Triage^{®}) for BNP assays, and between 10.9% (Vidas^{®}-Vitros^{®}) and 55% (CobasH232^{®}-Xpand^{®}) for NT-proBNP assays.

Collin-Chavagnac *et al.* concluded that their study stresses the lack of transferability of the results obtained using different techniques to measure BNP and NT-proBNP levels in fresh samples, adding that "individual reference ranges and HF diagnostic cut-offs should be assessed for each commercial NP immunoassay". The authors recommended that HF patients should be systematically monitored using the same assay (BNP or NT-proBNP) over the time (Collin-Chavagnac *et al., supra*).

### Example 2. Production of antibodies

Antibodies were produced in either a mammalian host (rabbit or goat), or in an avian host (hens) against different immunogens and purified as shown in Table 2 below.

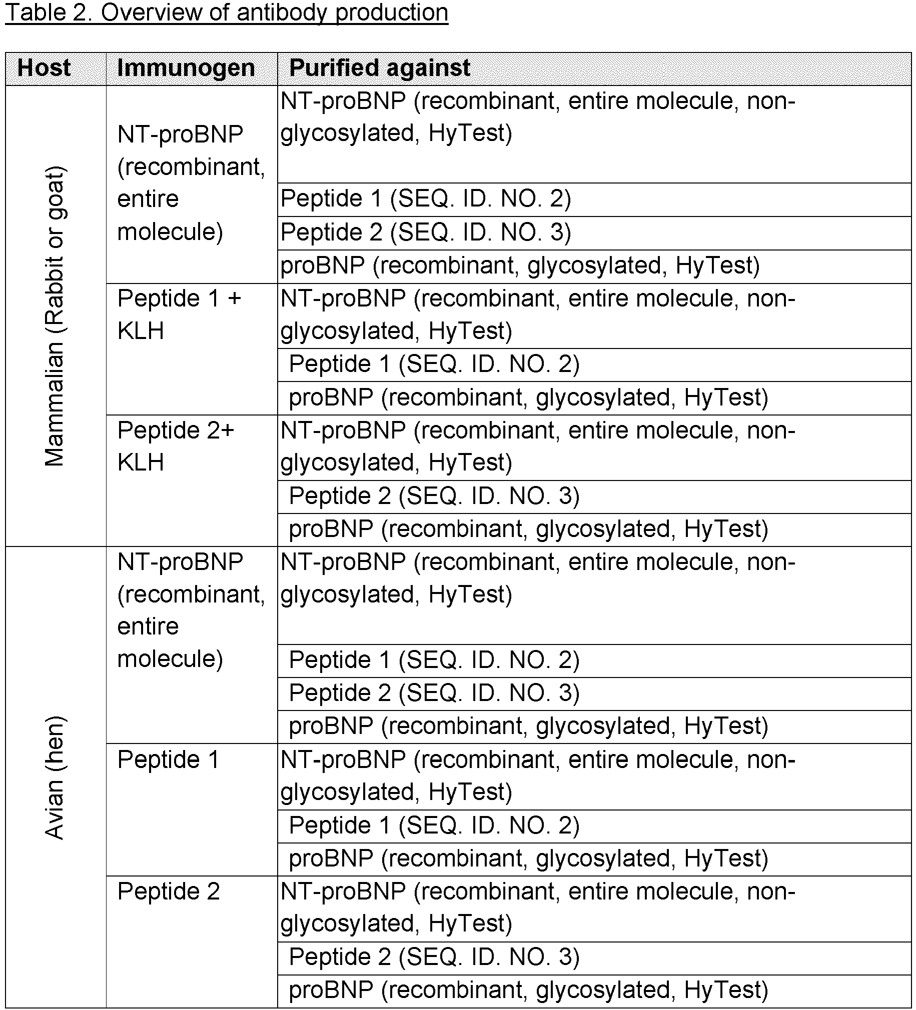

Antibodies against NT-proBNP and variants thereof (for example recombinant or native, truncated or non-truncated forms, glycosylated and non-glycosylated forms, and combinations thereof) can be produced according to methods known to a person skilled in the art, either in mammal hosts, e.g., goats or rabbits, or preferably in hens, in which case the antibodies can be isolated from the egg yolk. Two peptides designated "Peptide 1" and "Peptide 2" were synthetized (Genscript Biotech (Netherlands) B.V., Leiden, The Netherlands), delivered as a white lyophilized powder with > 90 % HPLC purity, and used as immunogens, in addition to the recombinant, full-length NT-proBNP in glycosylated and non-glycosylated form:
**HPLGSPGSAS DLETSGLQEQ RNHLQGKLSE LQVEQTSLEP LQESPRPTGV WKSREVATEG IRGHRKMVLY TLRAPR** (Full-length NT-proBNP, SEQ ID NO. 1)
**SAS DLETSGLQEQ RNHLQGKLSE LQV** (SEQ ID NO. 2, Peptide 1, amino acid residues 8 - 33 of SEQ ID NO. 1)
**IRGHRKMVLY TLRA** (SEQ ID NO. 3, Peptide 2, amino acid residues 61 - 74 of SEQ ID NO. 1)

When raising antibodies against a shorter peptide, such as Peptide 1 or Peptide 2, these are preferably conjugated to a suitable carrier to increase immunogenicity. One example is keyhole limpet hemocyanin (KLH) used in the immunization of the mammalian hosts (rabbits). Immunization and antibody purification protocols for raising polyclonal rabbit antibodies are well-known to a person skilled in the art, and available for example from CBG, Max Planck Institute of Molecular Cell Biology and Genetics. General procedures are also disclosed in Antibodies: A Laboratory Manual, Edward A. Greenfield (Ed.), Cold Spring Harbor Laboratory, Cold Spring Harbor, N.Y., 2012.

Avian immunoglobulin fractions such as those used in this assay can be purified from eggs from immunized hens, immunized with native or recombinant NT-proBNP or variants thereof, as indicated in Table 2. The antigen and suitable adjuvant, e.g., complete Freund's adjuvant, is injected subcutaneously and/or intramuscularly into breast tissue of the hen. Immunizations are repeated at regular intervals, for example on day 10, 20 and 30. Antibodies are usually detected in the eggs by day 30.

Eggs are collected, the yolk separated from the white, and the immunoglobulin fraction is isolated from the egg yolk. As a first step, the lipids and lipoproteins are removed, using a suitable method known to persons skilled in the art, for example precipitation using PEG or dextran sulphate, solubilisation using organic solvents, or ultrafiltration. The resulting substantially lipid-free solution can then be concentrated and purified to obtain the desired immunoglobulin fraction containing polyclonal antibodies against the antigen.

The immunisation of rabbits was performed by GenScript Biotech Corp., Piscataway, NJ, USA, and the immunization of hens by Norwegian Antibodies AS, NABAS, Ås, Norway. All antibodies were purified by Getica AB, Gothenburg, Sweden.

Goat IgG was produced by InnovaGen AB, Lund, Sweden, immunizing goats with a peptide according to SEQ ID NO. 2 and SEQ ID NO. 3, following the same methods, mutatis mutandis. Methods for the concentration and/or purification of immunoglobulins are also well known to a person skilled in the art and include precipitation steps using for example PEG or sodium sulphate, or other well-known methods such as ultrafiltration or liquid chromatography. For more guidance, see e.g. Antibodies: A Laboratory Manual, Edward A. Greenfield (Ed.), Cold Spring Harbor Laboratory, Cold Spring Harbor, N.Y., 2012.

Polyclonal antibodies against the peptides of SEQ ID NO 2 and SEQ ID NO. 3 also achieved high affinity (See Example 3 below) and high specificity, avoiding binding to glycosylated regions, which allows the measurement of the true NT-proBNP concentration with high sensitivity also in a turbidimetric or nephelometric setting.

### Example 3. Biacore^{™} affinity analysis

As part of the quality assurance in the production of antibodies for the experiments disclosed herein, affinity analysis was routinely performed for each batch. The affinity of an antibody for its antigen is determined by surface plasmon resonance (SPR) in a method often referred to as the Biacore^{™} method. SPR occurs under conditions of total internal reflection of plane-polarized light at an interface between two media of different refractive index, coated with an electrically conducting film. When a sample binds to the surface, the refractive index increases, and the resonance angle changes. The angle is monitored continuously as the SPR signal, and a plot of the signal against time, called a sensorgram, shows the changes in refractive index as a sample binds to and dissociates from the surface.

The analysis was performed using a Biacore X100 system, using the GE Sensor Chip CM5 (all from GE Healthcare). The chip has a gold surface with a dextran matrix having carboxyl groups on it. For determining the affinity of an antibody to an antigen, the surface of a chip is activated, and an antigen is covalently attached to the surface. This process is irreversible, so the chip can be re-used several times without the need for extra antigen.

For determining the kinetic constants, the target antibodies were first dialysed against PBS and then diluted in HBS to 30 nanomolar (nM). A dilution series was prepared (30 nM - 10 nM - 3.3 nM - 1.1 nM and 0.37 nM) for each target antibody. The concentration was confirmed by spectrophotometric analysis at 280 nm.

The antibodies were analysed on the Biacore X100 system, using the Biacore X100 Control Software, which when executed on the system injects the antibody dilution series in reverse (starting with the most diluted) sequentially and as a final step the surface is re-generated with glycine (pH 1.5). The kinetic constants are extracted from the sensorgram using the Biacore X100 Evaluation Software based on a 1:1 kinetic model. Typical results of the Biacore^{™} analysis are summarized in Table 3 below:

**Table 3. Dissociation constants**

| **Host** | **Immunogen** | **Purified against** | **Biacore^{™} data (dissociation constant, K_{D}, molar (M))** |
|---|---|---|---|
| Mammalian (rabbit) | Recombinant NT-proBNP, entire molecule | Recombinant NT-proBNP, entire molecule, non-glycosylated | 1.35E-09 |
| | | Peptide 1 | 8.67E-10 |
| | | Peptide 2 | 1.26E-09 |
| Avian (hen) | Recombinant NT-proBNP, entire molecule | Recombinant NT-proBNP, entire molecule, non-glycosylated | Average 5E-09 (several different lots tested) |
| | | Peptide 1 | 1.98E-09 |
| | | Peptide 2 | 1.72E-07 |
| | | proBNP (recombinant, glycosylated) | 5.24E-09 |

The results showed that the immunisation protocol and purification consistently produced antibodies binding specifically to NT-proBNP and exhibiting a K_{D} to recombinant NT-proBNP of less than 2.0E-09 M.

The affinity constants were plotted against the span response and the sensitivity of the assay when run on a clinical chemistry analyser (Mindray BS-400). This showed a clear correlation between span, sensitivity, and the dissociation constants.

IgG antibody against the peptide of SEQ ID NO 2 from goat antiserum, purified against the same peptide, was tested against a Biacore^{™} chip with the peptide of of SEQ ID NO 2. The equilibrium dissociation constant was determined to KD= 2.64E-09 M.

IgG antibody against the peptide of SEQ ID NO 3 from goat antiserum, purified against the same peptide, was tested against a Biacore^{™} chip with the peptide of of SEQ ID NO 3. The equilibrium dissociation constant was determined to KD= 9.72E-09 M.

Similarly, IgG antibody against the peptide of SEQ ID NO 3 from rabbit antiserum was tested against a Biacore^{™} chip with the peptide of of SEQ ID NO 3. The equilibrium dissociation constant was determined to KD= 6.54E-09 and 5.23E-09 M.

### Example 4. Epitope mapping

Epitope mapping was performed by Pepscan Presto BV, Lelystad, The Netherlands, using libraries of linear and looped peptide arrays synthesised onto a solid support covered with a hydrogel. The mapping technique was first published in 1984 (Geysen et al., Use of peptide synthesis to probe viral antigens for epitopes to a resolution of a single amino acid, PNAS, July 1, 1984 81 (13) 3998-4002). Chicken and rabbit antibodies were provided by Gentian AS, and the mapping focused on a 26 residues long segment of the NT-proBNP sequence, positions 8 - 33, sequence SASDLETSGLQEQRNHLQGKLSELQV (identical to SEQ ID NO. 2).

Synthesis of peptides: To reconstruct epitopes of the target molecule a library of peptide-based peptide mimics was synthesized using Fmoc-based solid-phase peptide synthesis. An amino functionalized polypropylene support was obtained by grafting with a proprietary hydrophilic polymer formulation, followed by reaction with t-butyloxycarbonyl-hexamethylenediamine (BocHMDA) using dicyclohexylcarbodiimide (DCC) with N-hydroxy-benzotriazole (HOBt) and subsequent cleavage of the Boc-groups using trifluoroacetic acid (TFA). Standard Fmoc-peptide synthesis was used to synthesize peptides on the amino-functionalized solid support by custom modified JANUS liquid handling stations (Timmerman et al., (2007) Functional reconstruction and synthetic mimicry of a conformational epitope using CLIPS™ technology, J. Mol. Recognit. 20:283-299; and Langedijk et al. (2011) Helical peptide arrays for lead identification and interaction site mapping, Analytical Biochemistry 417: 149-155).

In a first set, linear peptides of a length ranging from 5 - 24 derived from the target sequence /SEQ ID NO. 2) were synthetized with an offset of one residue. In a second set, constrained peptides of length 7 - 27 were synthetized starting from Linear peptides of length 5-25 residues derived from the target sequence of NT Pro BNP with an offset of one residue were placed between two Cys residues. Cys residues were joined by mP2 CLIPS in order to create a loop mimic.

The binding of antibody to each of the synthesized peptides was tested in an ELISA. The peptide arrays were incubated with primary antibody solution overnight at 4 °C. After washing, the peptide arrays were incubated with a 1/1000 dilution of an appropriate antibody peroxidase conjugate for one hour at 25 °C. After washing, the peroxidase substrate 2,2'-azino-di-3-ethylbenzthiazoline sulfonate (ABTS) and 20 µl/ml 3 % H₂O₂ were added. After one hour, the color development was measured. The color development was quantified with a charged coupled device (CCD) -camera and an image processing system.

The main epitope candidates identified in this study are presented in the table below, as well as in the enclosed sequence listing:

**Table 4. Epitope candidates based on epitope mapping**

| **Amino acid sequence** | **SEQUENCE ID. NO.** |
|---|---|
| SASDL | SEQ. ID. NO. 4 |
| DLETSGLQEQRNHL | SEQ. ID. NO. 5 |
| NHLQGKL | SEQ. ID. NO. 6 |
| SASDLETS | SEQ. ID. NO. 7 |
| ETSGLQ | SEQ. ID. NO. 8 |
| GKLSELQ | SEQ. ID. NO. 9 |
| GLQEQR | SEQ. ID. NO. 10 |

The epitope mapping confirmed that the antibodies bind to the correct portions of the NT-proBNP molecule.

### Example 5. Preparation of magnetic affinity beads

Superparamagnetic beads with a diameter of 2.8 µm containing surface epoxy groups were chosen for this example. 10 mg Dynabeads^{™} M-270 Epoxy were weighed out in a tube. 500 µL distilled water was added. The mixture was vortexed for 30 sec and sonicated with a sonication probe for 20 seconds. Supernatant was removed by placing the tube on a magnetic rack. This was repeated once.

Goat anti-NT-proBNP antibodies and rabbit anti-NT-proBNP antibodies (produced as shown in Example 2) were coated, separately, onto the Dynabeads^{®}. The same coating procedure was used for both batches. The antibodies were buffer exchanged into 0.1 M sodium phosphate buffer, pH 7.4 before coating. 100 µg antibody in a total of 250 µL 0.1 M sodium phosphate buffer, pH 7.4 was reacted to the beads. The beads were vortexed for 5 sec before 250 µL 3 M ammonium sulphate [(NH₄)₂SO₄] in 0.1 M sodium phosphate buffer pH 7.4 was added. The mixture was vortexed for 5 sec and incubated with mixing at 37°C overnight.

The particle mixture was mixed on a roller mixer for 3 hours in room temperature, before the supernatant was removed, and the beads were washed in 500 µL 100 mM glycine pH 11.3 buffer, vortexed for 5 sec, separated on a magnet (1 min) and supernatant removed. The beads were then washed in 500 µL 200 mM Glycine pH 2.8, and the same procedure followed. Then 500 µL 15 mMTris-HCl buffer, 140 mM NaCl pH 7.6 was added to the beads, the beads were placed on a roller mixer for 15 min, separated on a magnet (1 min) and supernatant removed. Finally, 500 µL 15mM Tris-HCl buffer, 140 mM NaCl pH 7.6 was added to the beads, the beads were placed on a mixer and incubated at 37 °C overnight. The following morning, the beads were put on a roller mixer for 8 hours, separated on a magnet (1 min) and supernatant removed. The Dynabeads^{®} were suspended in 15 mM Tris-HCl buffer, 140 mM NaCl pH 7.6, to a final concentration of 10 mg/mL

### Example 6. Preparation of standards

The calibrator matrix can be constituted by serum or plasma materials from human or animal serum or plasma materials, or a buffer solution, comprising a known amount of the antigen, which can be human, recombinant, or synthetic NT-proBNP, or fragments thereof, such as peptide 1 (SEQ ID NO 2), Peptide 2 (SEQ ID NO 3), optionally conjugated to a carrier protein. The calibrator material can further comprise a salt solution, soluble polymers such as PEG-6000 (or other PEG materials) and preservative agents.

The composition of the calibrator can be adjusted until the assay method generates results corresponding to values obtained using a reference method as described herein, where the concentration of the calibrator is assigned by an immunoassay-based method using an antibody which binds specifically to a non-glycosylated epitope on the NT-proBNP molecule.

The concentration assignment of the calibrator material is well described in the prior art, for example in Blirup-Jensen, S. et al., Protein Standardization IV: Value Transfer Procedure for the Assignment of Serum Protein Values from a Reference Preparation to a Target Material, Clin Chem Lab Med 2001; 39(11): 1110-1122.

Recombinant NT proBNP (8NT2, HyTest) was trypsinated in solution; 25 µl of NT proBNP standard in 100 mM ammonium bicarbonate was denatured with 25 µl Trifluoroetanol (TFE), reduced with 2.5 µl 200 mM dithiothreitol (DTT) at 90 °C for 20 min; and alkylated with 10 µl 200 mM iodoacetamide (IAA) at room temperature in the dark for 1 h. Thereafter 2.5 µl 200 mM DTT was added to remove excess IAA, then the mixture was kept in room temperature in the dark for 1 h.

TFE concentration was diluted 6-fold with 25 mM ammonium bicarbonate. Trypsin (sequencing grade, Promega) was diluted in ammonium bicarbonate and added at a protein to enzyme ratio of 1:35. Digestion was performed overnight at 37 °C, stopped by addition of 2 µl formic acid after which the resulting peptides were desalted on C18 SPE columns.

Isotope labelled (deuterium labelled) peptide standards were ordered from ThermoFisher following instructions from the inventors. Heavy peptide NHLQG(K) (SEQ ID NO. 11) contained labelled Lysine (+8 Da), heavy peptide EVATEGI(R) (SEQ ID NO. 12) contained labelled Arginine (+10 Da), and heavy peptide MVLYTL(R) (SEQ ID NO. 13) contained labelled Arginine (+10 Da). Alternatively, heavy-labelled (15N) full-length NTproBNP from Promise Proteomics, Grenoble Cedex, France, was used for calibration and sample control.

The desalted peptides and labelled peptide standards were dried to dryness via vacuum centrifugation and stored frozen.

A plasma or a serum sample is a highly complex environment, containing multiple biologically active components, and additionally, as can be seen in Fig. 1, BNP has many molecular forms. The identification of three specific peptide standards makes it possible to use one as the main standard for quantification, and one or two for a secondary, qualitative confirmation that it actually is NT-proBNP that is detected and quantified.

### Example 7. Sample preparation by affinity capture of NT-proBNP and subsequent enzymatic digestion

Sample preparation was performed by affinity purification of NT-proBNP from plasma/serum samples using magnetic affinity beads (Dynabeads^{®}), prepared as described above in Example 4. The beads were washed in PBS prior to use. Both rabbit and goat antibody carrying beads were used (separately).

Plasma samples were processed as follows; plasma (0.5 ml) was mixed with 25 µl Dynabeads^{®}, placed on mixer and incubated over-night at 4 °C. Following incubation, the beads were placed in a magnetic rack and the supernatant was removed. The beads were thereafter washed in PBS (V = 1 ml x 2) and thereafter washed in 1 ml 50 mM ammonium bicarbonate.

Trypsination was performed on-bead in 100 µl 50 mM ammonium bicarbonate following a previously described protocol [Levernæs et al., To elute or not to elute in immunocapture bottom-up LC-MS, J Chromatogr B Analyt Technol Biomed Life Sci. 2017;1055-1056:51-60] with some modifications; NT-proBNP attached to affinity beads (100 µl of beads in ammonium carbonate) was reduced with 5 µl 200 mM dithiothreitol (DTT) at 60 °C, 800 rpm for 15 min; and alkylated with 15 µl 200 mM iodoacetamide (IAA) at room temperature in the dark for 15 min. Thereafter 5 µl 200 mM DTT was added to remove excess IAA. Trypsin (sequencing grade, Promega) was diluted in ammonium bicarbonate and added in excess, at a ratio guaranteeing that all protein is degraded.

Digestion was performed overnight at 37 °C. The tubes with magnetic beads were placed in the magnetic rack and the tryptic peptide solutions were transferred to new tubes and centrifuged at 5000 rpm for 2 min to sediment any remaining trace particles. Tryptic peptide solutions were transferred to new tubes and 2 µl formic acid was added. The resulting peptides were desalted on C18 SPE columns.

### Example 8. Peptide clean-up

The resulting peptides (from both standards and affinity samples) prepared as shown in Example 6, were then desalted on C18 spin columns (C 18 Spin Columns (Pierce, Rockford, USA)). The C18 spin columns were then washed with 2x200 µL 50 % ACN and conditioned with 2x200 µL 0.5 % TFA in 5 % ACN prior to use. After sample (<30 ug total protein) was added to the spin columns, they were washed with 2x200 µL 0.5 % TFA in 5 % ACN to remove salt. Peptides were eluted in two portions of 20 µL 70 % ACN with 0.1 % FA. Eluates were dried to dryness via vacuum centrifugation and stored frozen until reconstitution and analysis.

Alternatively, the peptides from both standards and samples were desalted on C18 Bond Elut LRC-C18 SPE columns (Agilent Technologies Inc., USA). The columns were washed with 2 ml 50 % ACN and conditioned with 2 ml 5 % ACN with 0.5 % TFA prior to use. After sample (400 µl trypsinated peptides plus 0.8 ml H2O) was added to the spin columns, they were washed with 1 ml 5 % ACN with 0.5 % TFA to remove salt and allowed to dry for 3 to 5 minutes. Peptides were eluted either in four portions of 0.5 ml or two portions of 0.7 ml 70 % ACN with 0,1 % FA. Eluates were dried to dryness via vacuum centrifugation and stored frozen until reconstitution and analysis.

### Example 9. Performing LC-MS/MS analysis

For an overview of immunocapture bottom-up liquid chromatography mass spectrometry, see e.g. Pitt, James J., Principles and Applications of Liquid Chromatography Mass Spectrometry in Clinical Biochemistry, Clin Biochem rev, 2009 Feb ; 30(1): 19-34, and Stillesby Levernaes et al., To elute or not elute in immunocapture bottom-up LC MS, Journal of Chromatography B, Volumes 1055-1056, 15 June 2017, Pages 51-60.

This analysis method included the following three peptides: NHLQG(K) (SEQ ID NO. 11), EVATEGI(R) (SEQ ID NO. 12), and MVLYTL(R) (SEQ ID NO. 13)

The corresponding heavy peptides were used for quantitation. The signals for oxidized peptides were also monitored in the method.

20 samples for LC-MS/MS analysis were reconstituted in 25 µl of 20% ACN with the addition of heavy peptides. The heavy peptides were added to concentrations of 0.005 pmol/µl for EVATEGIR/MVLYTR (SEQ ID NO:s 5 and 6) and 0,05 pmol/µl NHLQGK (SEQ ID NO 4).

When using 15N full length NT-proBNP, the standard was added to the plasma prior to the affinity purification on beads and trypsinated together with the samples.

The LC-MS analyses were performed on an Agilent 1200 Series LC/MS system (Agilent Technologies Inc., Palo Alto, CA, USA). The system comprised a G4220A binary pump, a G4226A autosampler, a G1316C column thermostat and a G6490 triple quadrupole mass spectrometer.

The LC-MS analysis was performed in reversed phase mode employing an Ascentis Express Phenyl Hexyl (2.1 x 150 mm, 2.1 µm) and gradient elution using 25 mM formic acid as eluent A and acetonitrile as eluent B. The gradient started at 1% eluent B for 1.8 min after which percentage B was increased to 65% over 3.7 minutes, held constant at 65 % for 1 minute and thereafter returned to the starting conditions for system equilibration. The flowrate was 0.3 ml/min and the column temperature was maintained at 20 °C. All analyses were performed with electro spray ionization (ESI) in positive mode. Mass spectrometric detection was performed in multiple reaction monitoring (MRM) mode. See Table 5 for the MRM-settings used.

**Table 5. Signature peptides and MRM-settings**

| **Signature peptide** | **Type** | **Precursor ion (m/z)** | **Product ion (m/z)** | **Dwell** | **CE (V)** | **Cell acc. (V)** |
|---|---|---|---|---|---|---|
| NHLQG(K) | light | 348.7 | 445.1 | 50 | 8 | 2 |
| | | 348.7 | 340 | 50 | 8 | 2 |
| | | 348.7 | 291.8 | 50 | 12 | 5 |
| MVLYTL(R) | light-ox* | 456.3 | 665.4 | 20 | 16 | 7 |
| | | 456.3 | 552.3 | 20 | 8 | 2 |
| | | 456.3 | 424.2 | 20 | 12 | 4 |
| | 15N-heavy | 453.4 | 773.4 | 20 | 8 | 3 |
| | | 453.4 | 673.4 | 20 | 8 | 7 |
| | | 453.4 | 559.3 | 20 | 16 | 3 |
| | light | 448.4 | 764.5 | 20 | 8 | 3 |
| | | 448.4 | 665.5 | 20 | 8 | 7 |
| | | 448.4 | 552.3 | 20 | 16 | 2 |
| EVATEGI(R) | 15N-heavy | 443.2 | 655.3 | 20 | 14 | 2 |
| | | 443.2 | 583.3 | 20 | 14 | 2 |
| | | 443.2 | 481.2 | 20 | 8 | 3 |
| | light | 437.8 | 646.4 | 20 | 14 | 2 |
| | | 437.8 | 575.8 | 20 | 14 | 2 |
| | | 437.8 | 474.3 | 20 | 8 | 3 |

Abbreviations: CE = collision energy, Cell acc = cell acceleration voltage. Instrument settings: Fragmentor 380 V. Gas Temp (°C): 150, Gas Flow (I/min): 20, Nebulizer (psi): 50, Sheath Gas Temp (°C): 300, Sheath Gas Flow (l/min): 11, Capillary (V) 3500, Nozzle Voltage/Charging (V): 0.

*Oxidised peptides. Monitored to assess oxidation during sample work-up. No specific standards available.

Three tryptic peptides were monitored, NHLQG(K), EVATEGI(R) and MVLYTL(R) (SEQ ID Nos 11, 12, and 13 respectively) along with the corresponding heavy-peptides (heavy-N labelled) originating from the internal standard N15-labelled NT-proBNP. For peptide MVLYTL (R), MRM transitions for an oxidised variant of the peptide and corresponding oxidised version of the heavy-labelled peptide were also monitored in the method. Multiple transitions were monitored for each peptide with the most abundant being used for quantitation and additional transitions used for qualification. Trypsinated standards of NT-pro-BNP at 0.1-1000 ng/ml were used in calibration.

### Data Analysis and Quantitation

Data analysis was performed using Agilent MassHunter Qualitative and Quantitative analysis software. Quantitation was based on calibration against trypsinated standard NT-proBNP spike in in plasma at 0-4-8-16-24-32 ng/ml. Sample quantitation was also performed based on standard addition results. All standards and samples were spiked with 24 ng/ml of internal standard (15N-labelled NTproBNP). Three peptides were monitored (NHLQG(K), EVATEGI(R) and MVLYTL(R)). Heavy peptides (15N-labelled from 15N-labelled NTproBNP) were used as internal standards. Peptides EVATEGI(R) and MVLYTL (R) were shown to have the best response. They exhibited LOD/LOQ corresponding to 1 and 0.5 ng/ml NT-ProBNP and linear response to 500 ng/ml. Quantitation was done based on the EVATEGI(R) peptide (SEQ ID NO. 12). Multiple transitions were monitored for each peptide with the most abundant being used for quantitation and additional transitions used for qualification, i.e., the verification that the correct/selected peptide is measured (and not an unrelated peptide with the same m/z). Here, peptide NHLQG(K) (SEQ ID NO. 11) was only used for qualification.

The results from evaluating a pool of 20 samples are presented in Table 6 below, where the results obtained with a prior art method are compared to the results obtained with the method disclosed in the present application. The last column shows the ratio between the results, indicating that the disclosed in the present application consistently result in a higher reading, and that the difference is more pronounced at lower NT-proBNP concentrations.

**Table 6. Results of LC-MS/MS analysis of NT-proBNP compared to prior art analysis**

| **Sample #** | **Prior art method [pg/ml]** | **Method according to the present invention [pg/ml]** | **Ratio** |
|---|---|---|---|
| 1 | 127 | 785 | 6,2 |
| 2 | 133 | 993 | 7,5 |
| 3 | 155 | 993 | 6,4 |
| 4 | 222 | 1729 | 7,8 |
| 5 | 350 | 2511 | 7,2 |
| 6 | 480 | 2622 | 5,5 |
| 7 | 519 | 3017 | 5,8 |
| 8 | 669 | 5069 | 7,6 |
| 9 | 698 | 3693 | 5,3 |
| 10 | 761 | 4260 | 5,6 |
| 11 | 787 | 4097 | 5,2 |
| 12 | 905 | 4764 | 5,3 |
| 13 | 1746 | 8079 | 4,6 |
| 14 | 1982 | 9602 | 4,8 |
| 15 | 3097 | 14313 | 4,6 |
| 16 | 3119 | 9556 | 3,1 |
| 17 | 4351 | 11730 | 2,4 |
| 18 | 5889 | 16261 | 2,0 |
| 19 | 6526 | 17291 | 2,6 |
| 20 | 13226 | 24498 | 1,9 |

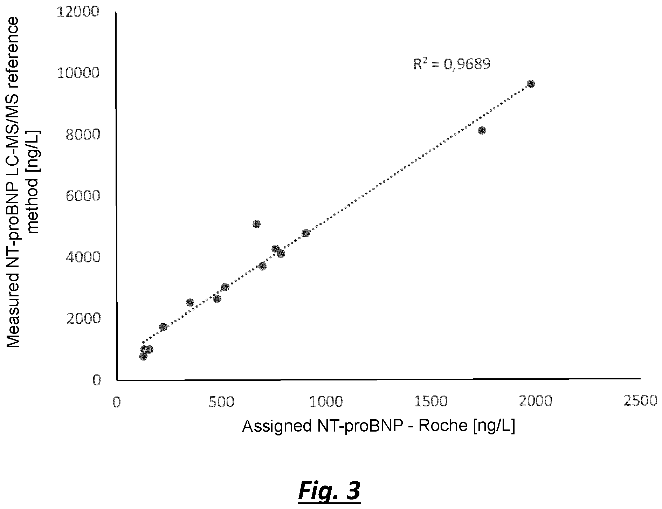

Within the clinically relevant range, 100 - 2000 ng/L as determined by a prior art method (the Roche Elecsys^{®} NT-proBNP assay), the results exhibit a linear correlation (R² = 0.9689) as shown in Figure 3. The graph shows that the commercial assay consistently underestimated the concentration of NT-proBNP in the mentioned concentration range and that the comparison exhibits a linear correlation (R2=0.9689).

For all 20 samples, in an interval of 100 - 14000 ng/L as determined by the prior art method, the correlation is still very good (R² = 0.9622).

For three samples, the stability of the method was tested by performing analysis of the same samples on two consecutive days. The results are presented in Table 7:

**Table 7. Analysis results Day 1 vs. Day 2**

| **Day** | **Sample #** | **Prior art method [pg/ml]** | **Method according to the present invention [pg/ml]** | **Ratio** | **Ratio Day 1 vs. Day 2** |
|---|---|---|---|---|---|
| 1 | 1 | 669 | 5069 | 7.6 | 1.01 |
| | 2 | 6526 | 17291 | 2.6 | 1.12 |
| | 3 | 13226 | 24498 | 1.9 | 0.91 |
| 2 | 1 | 669 | 4998 | 7.5 | |
| | 2 | 6526 | 15387 | 2.4 | |
| | 3 | 13226 | 26940 | 2.0 | |

As can be seen from the results, the method was reproducible and stable in the clinically relevant range.

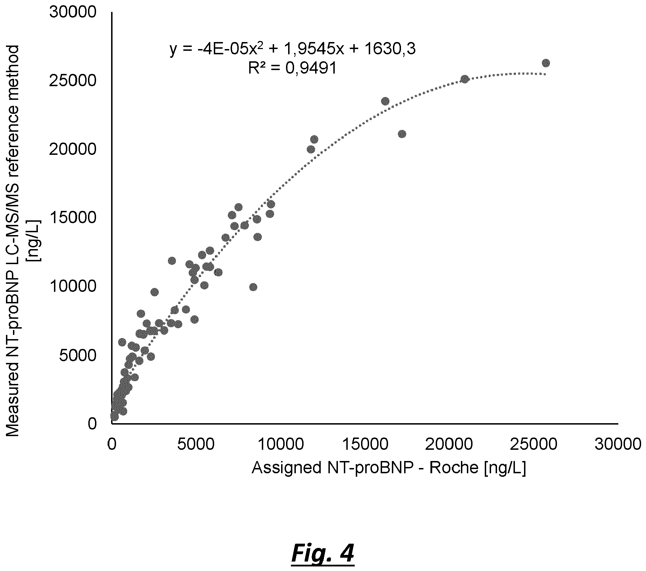

During the priority year, a larger number of samples covering a wider concentration range (0 to 25000 ng/L) were investigated, and a comparison made between the results obtained with the claimed method and a commercial assay, the Roche Elecsys^{®} NT-proBNP assay, performed according to the manufacturer's instructions. The results are shown in Figure 4. This graph confirms the results presented in Figure 3 but also reveals that the concentration measurements between the commercial assay and the herein presented method converge in the higher concentrations. Both methods exhibit a good (polynomial) fit over a large concentration range (R² = 0.9491).

### Example 10. Comparative analysis of plasma and serum samples

To investigate the utility of the inventive method, both plasma and serum samples were analysed using on the one hand the Siemens ADVIA Centaur^{®} Immunoassay System (for plasma samples) and on the other hand the Siemens IMMULITE Immunoassay System (for serum samples). Both assays were performed according to the manufacturer's instructions. The results were compared to the concentrations determined using the herein claimed LC-MS/MS method.

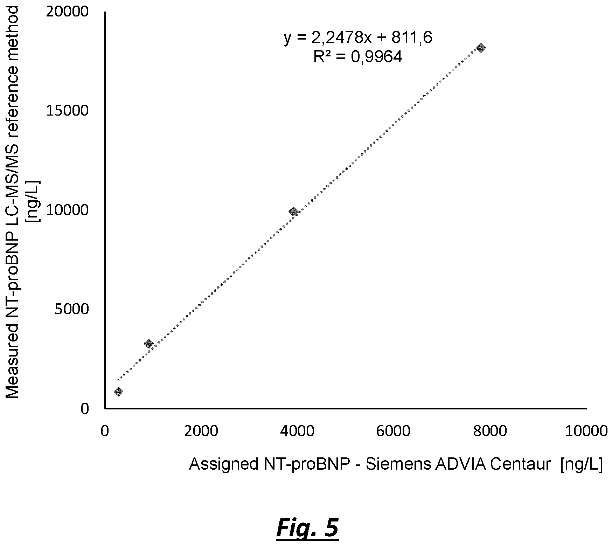

Figure 5 shows that the Siemens ADVIA assay underestimated the concentration of NT-proBNP in the lower concentration range, but that the comparison with concentrations determined using the claimed method exhibits a linear correlation (R2=0.9964).

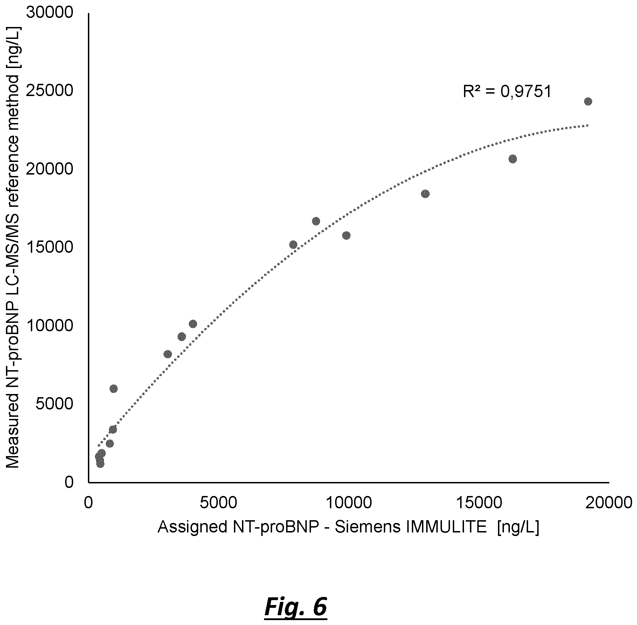

Figure 6 shows the result for serum samples using another commercial method, the Siemens IMMULITE. The graph confirms the results presented in Figure 5, showing again an underestimation of the NT-proBNP concentration in the lower concentration range by the commercial assay, but also a convergence of results between the commercial and the herein presented method in the higher concentrations for different sample types. Both methods exhibit good (polynomial) fit over a larger concentration range (R2 = 0.9751).

### Example 11. Demonstrating the robustness of method

The method disclosed herein was set up at two geographically different sites here denoted "1" and "2" and two operators at each site were trained to perform the analysis. At the first site, the measurements were made using a LC-ESI-Triple Quadrupole instrument (Agilent, USA) which is a liquid chromatography - electrospray ionization tandem mass spectrometer consisting of two quadrupole mass analysers in series, with a (non-mass-resolving) radio frequency-only quadrupole between them to act as a cell for collision-induced dissociation.At the second site, a nano-LC-ESI-Orbitrap instrument (Thermo Fisher Scientific, USA) was used.

In an initial comparison, 4 plasma samples with distinct concentrations (determined using the Roche method) were measured at both sites, and the measurements were made independently by two operators at each site. The operators were denoted A, B, C and D. The results are shown in Table 8.

**Table 8. Comparison of method performance at two sites**

| **Sample** | **Site 1 "A"** | **Site 1 "B"** | **Site 2 "C"** | **Site 2 "D"** | **Average** | **CV** |
|---|---|---|---|---|---|---|
| **(ng/L)** | **(ng/L)** | **(ng/L)** | **(ng/L)** | **(ng/L)** | **(ng/L)** | **(%)** |
| 220 | 990 | 1060 | 780 | 870 | 925 | 13 |
| 1050 | 3150 | 3490 | 3100 | 3080 | 3205 | 6 |
| 2620 | 6930 | 6940 | 6740 | 6740 | 6838 | 2 |
| 9700 | 13460 | 13270 | 12960 | 13080 | 13193 | 2 |

Overall, the results indicate that the herein disclosed assay is robust, stable, and capable of detecting the total amount of NT-proBNP in a sample, i.e. that the method detects NT-proBNP independent of its degree of glycosylation.

The inventors have now shown, based on the isolation and quantification of NT-proBNP using different immunoassay-based reference methods, involving the use of antibodies specifically binding to non-glycosylated epitopes of NT-proBNP both for determining the NT-proBNP concentration in the calibrator sample and in the test or patient sample, that a significantly higher concentration of NT-proBNP is detected in the test or patient sample. This is held to be the "true" concentration of NT-proBNP, independent of degree of glycosylation and other variables. Thus, a more reliable, and glycosylation-independent assay principle has been developed and its utility and robustness has been confirmed.

The method disclosed herein can be used to calibrate / validate existing assays or used as a stand-alone method in clinical chemistry. This will enable a more reliable and correct diagnosis of heart failure, in particular congestive heart failure. Further, as it has been shown that this approach is applicable to turbidimetry and nephelometry, this opens up the possibility of automated, cost-efficient and yet reliable methods for diagnosing of heart failure or an elevated risk for heart failure, monitoring a subject with heart failure or having an elevated risk for heart failure, or assessing the severity of heart failure, by determining the concentration of NT-proBNP in a bodily fluid sample of said subject suspected of having heart failure.

Without further elaboration, it is believed that a person skilled in the art can, using the present description, including the examples, utilize the present invention to its fullest extent. Also, although the invention has been described herein with regard to its preferred embodiments, which constitute the best mode which is set forth in the claims appended hereto.

Thus, while various aspects and embodiments have been disclosed herein, other aspects and embodiments will be apparent to those skilled in the art. The various aspects and embodiments disclosed herein are for purposes of illustration and are not intended to be limiting.

### References

Blirup-Jensen, S. et al., Protein Standardization IV: Value Transfer Procedure for the Assignmnt of Serum Protein Values from a Reference Preparation to a Target Material, Clin Chem Lab Med 2001; 39(11): 1110-1122)
Collin-Chavagnac et al., Head-to-head comparison of 10 natriuretic peptide assays, Clinical Chemistry and Laboratory Medicine, April 2015, Doi: 10.1515/cclm-2014-0592
Edward A. Greenfield (Ed.), Antibodies: A Laboratory Manual, Cold Spring Harbor Laboratory, Cold Spring Harbor, N.Y., 2012.
Geysen et al., Use of peptide synthesis to probe viral antigens for epitopes to a resolution of a single amino acid, PNAS, July 1, 1984 81 (13) 3998-4002
Halfinger, B. et al., Unravelling the Molecular Complexity of O-Glycosylated Endogenous (N-Terminal) pro-B-Type Natriuretic Peptide Forms in Blood Plasma of Patients with Severe Heart Failure, Clinical Chemistry, 2017, Vol. 63, 1
Harlow et al. (Eds.), Antibodies: A Laboratory Manual; Cold Spring Harbor Laboratory; Cold Spring Harbor, NY (1988), Chapter 6
Januzzi et al., NT-proBNP testing for diagnosis and short-term prognosis in acute destabilized heart failure: an international pooled analysis of 1256 patients. Eur Heart J 2006; 27: 330-37
Langedijk et al. (2011) Helical peptide arrays for lead identification and interaction site mapping, Analytical Biochemistry 417:149-155
McKie and Burnett, NT-proBNP: The Gold Standard Biomarker in Heart Failure, J Am Coll Cardiol, 2016 Dec 6;68(22):2437-2439Langedijk et al. (2011) Helical peptide arrays for lead identification and interaction site mapping, Analytical Biochemistry 417:149-155
Pitt, James J., Principles and Applications of Liquid Chromatorgraphy Mass Spectrometry in Clinical Biochemistry, Clin Biochem rev, 2009 Feb ; 30(1): 19 34
Røsjø et al., Influence of glycosylation on diagnostic and prognostic accuracy of N-terminal pro-B-type natriuretic peptide in acute dyspnea: data from the Akershus Cardiac Examination 2 Study, Clin Chem, 2015 Aug;61(8):1087-97. doi: 10.1373/clinchem.2015.239673. Epub 2015 Jun 8.
Saenger, A. K. et al., Specificity of B-Type Natriuretic Peptide Assays: Cross-Reactivity with Different BNP, NT-proBNP, and proBNP Peptides, Clinical Chemistry, 63:1 (2017) 351-358
Schellenberger, U. et al., The precursor to B-type natriuretic peptide is an O-linked glycoprotein, Arch Biochem Biophys. 2006 Jul 15;451(2):160-6. Epub 2006 Apr 19
Semenov A.G. and Feygina, E.E., Standardization of BNP and NT-proBNP Immunoassays in Light of the Diverse and Complex Nature of Circulating BNP-Related Peptides, Advances in Clinical Chemistry, Volume 85, pages 1-30, https://doi.org/10.1016/bs.acc.2018.02.001
Stillesby Levernaes et al., To elute or not elute in immunocapture bottom-up LC MS, Journal of Chromatography B, Volumes 1055-1056, 15 June 2017, Pages 51-60
TechNotes | Human ProBNP, BNP and NT-proBNP, HyTest Ltd., January 2019, https://shop.hytest.fi/spree/products/2932/Human_proBNP_BNP_and _NT-proBNP_TechNotes.pdf?1560757320, downloaded 07.08.2019
Timmerman et al., (2007) Functional reconstruction and synthetic mimicry of a conformational epitope using CLIPS™ technology, J. Mol. Recognit. 20:283-299

## Claims

1. A method for determining a concentration of human N-terminal pro-hormone BNP (NT-proBNP) in a sample, **characterized in that** the method comprises
- extracting and isolating NT-proBNP from the sample using an affinity purification method making use of affinity beads carrying at least one antibody against said NT-proBNP, and wherein said at least one antibody exhibits high affinity for a non-glycosylated epitope on the NTproBNP molecule,
- adding a least one labelled peptide standard to said isolated NT-proBNP,
- subjecting said isolated NT-proBNP and peptide standard to enzymatic digestion wherein said enzymatic digestion is a trypsinization, and wherein said trypsinization is performed while the labelled standard and native NT-proBNP are associated with said affinity beads, producing a mixture of peptides, and
- detecting at least one peptide in the resulting mixture, using liquid chromatography-mass spectrometry analysis with subsequent mass spectrometric detection, wherein said at least one peptide to be detected is chosen from NHLQG(K) (SEQ ID NO. 11), EVATEGI(R) (SEQ ID NO. 12), and MVLYTL(R) (SEQ ID NO. 13).

2. The method according to claim 1, wherein said at least one labelled peptide standard is full-length recombinant NT-proBNP.

3. The method according to claim 1, wherein said at least one peptide standard is chosen from
NHLQG(K) (SEQ ID NO. 11),
EVATEGI(R) (SEQ ID NO. 12), and
MVLYTL(R) (SEQ ID NO. 13).

4. The method according to claim 3, wherein said at least one peptide standard is EVATEGI(R) (SEQ ID NO. 12).

5. The method according to claim 3, wherein said at least one peptide standard is a deuterium labelled EVATEGI(R) (SEQ ID NO. 12).

6. The method according to claim 1, wherein said affinity beads are magnetic affinity beads.

7. The method according to claim 1, wherein said at least one antibody binds specifically to a non-glycosylated epitope on the NT-proBNP within an amino acid sequence chosen from SEQ ID NO. 2 and SEQ ID NO. 3.

8. The method according to claim 1, wherein quantitation is performed based on calibration against enzymatically digested recombinant NT-proBNP.

9. The method according to claim 1, wherein the method is calibrated using trypsinated standard NT-proBNP at relevant concentrations, and wherein all standards are spiked with an internal standard.

10. The method according to claim 9, wherein the internal standard is labelled recombinant full-length NT-proBNP.

11. A method according to any one of claims 1 - 10 for use in assigning values to calibration materials, testing and/or quality control of assays for determining a concentration of human N-terminal pro-hormone BNP (NT-proBNP) in a sample.

12. A method according to any one of claims 1 - 10 for use in the diagnosis and monitoring of heart failure.

## Patentansprüche

1. Verfahren zur Bestimmung einer Konzentration des menschlichen N-terminalen Pro-Hormons BNP (NT-proBNP) in einer Probe, **dadurch gekennzeichnet, dass** das Verfahren umfasst
- Extrahieren und Isolieren von NT-proBNP aus der Probe unter Verwendung eines Affinitätsreinigungsverfahrens, bei dem Affinitäts-Beads verwendet werden, die mindestens einen Antikörper gegen das NT-proBNP tragen, und wobei der mindestens eine Antikörper eine hohe Affinität für ein nicht-glykosyliertes Epitop auf dem NTproBNP-Molekül aufweist,
- Zugeben mindestens eines markierten Peptidstandards zu dem isolierten NT-proBNP,
- Enzymatisches Verdauen des isolierten NT-proBNP und des Peptidstandards, wobei der enzymatische Verdau eine Trypsinierung ist und wobei die Trypsinierung durchgeführt wird, während der markierte Standard und das native NT-proBNP mit den Affinitäts-Beads assoziiert sind, wodurch ein Peptidgemisch erzeugt wird, und
- Nachweisen mindestens eines Peptids in dem resultierenden Gemisch unter Verwendung einer Flüssigchromatographie-Massenspektrometrie-Analyse mit anschließendem massenspektrometrischen Nachweis, wobei das mindestens eine nachzuweisende Peptid ausgewählt ist aus NHLQG(K) (SEQ ID NO. 11), EVATEGI(R) (SEQ ID NO. 12) und MVLYTL(R) (SEQ ID NO. 13).

2. Verfahren nach Anspruch 1, wobei der mindestens eine markierte Peptidstandard rekombinantes Volllängen-NT-proBNP ist.

3. Verfahren nach Anspruch 1, wobei der mindestens eine Peptidstandard ausgewählt ist aus
NHLQG(K) (SEQ ID NO. 11),
EVATEGI(R) (SEQ ID NO. 12) und
MVLYTL(R) (SEQ ID NO. 13).

4. Verfahren nach Anspruch 3, wobei der mindestens eine Peptidstandard EVATEGI(R) (SEQ ID NO. 12) ist.

5. Verfahren nach Anspruch 3, wobei der mindestens ein Peptidstandard ein Deuterium-markierter EVATEGI(R) (SEQ ID NO. 12) ist.

6. Verfahren nach Anspruch 1, wobei die Affinitäts-Beads magnetische Affinitäts-Beads sind.

7. Verfahren nach Anspruch 1, wobei der mindestens eine Antikörper spezifisch an ein nicht-glykosyliertes Epitop auf dem NT-proBNP innerhalb einer Aminosäuresequenz, ausgewählt aus SEQ ID NO. 2 und SEQ ID NO. 3, bindet.

8. Verfahren nach Anspruch 1, wobei die Quantifizierung auf der Grundlage einer Kalibrierung gegen enzymatisch verdautes rekombinantes NT-proBNP durchgeführt wird.

9. Verfahren nach Anspruch 1, wobei das Verfahren unter Verwendung von trypsiniertem Standard-NT-proBNP in relevanten Konzentrationen kalibriert wird und wobei alle Standards mit einem internen Standard gespiked werden.

10. Verfahren nach Anspruch 9, wobei der interne Standard markiertes, rekombinantes Volllängen-NT-proBNP ist.

11. Verfahren nach einem der Ansprüche 1 bis 10 zur Verwendung bei der Zuordnung von Werten zu Kalibrierungsmaterialien, beim Testen und/oder bei der Qualitätskontrolle von Assays zur Bestimmung der Konzentration des menschlichen N-terminalen Pro-Hormons BNP (NT-proBNP) in einer Probe.

12. Verfahren nach einem der Ansprüche 1 bis 10 zur Verwendung bei der Diagnose und Überwachung von Herzinsuffizienz.

## Revendications

1. Procédé de détermination d'une concentration d'une pro-hormone BNP N-terminale humaine (NT-proBNP) dans un échantillon, **caractérisé en ce que** le procédé comprend les étapes consistant à
- extraire et isoler la NT-proBNP de l'échantillon en utilisant un procédé de purification d'affinité utilisant des billes d'affinité portant au moins un anticorps contre ladite NT-proBNP et ledit au moins un anticorps présentant une haute affinité pour un épitope non glycosylé sur la molécule de NT-proBNP,
- ajouter au moins un peptide marqué standard à ladite NT-proBNP isolée,
- soumettre ladite NT-proBNP isolée et le peptide standard à une digestion enzymatique ladite digestion enzymatique étant une trypsinisation, et ladite trypsinisation étant effectuée alors que le standard marqué et la NT-proBNP native sont associés avec lesdites billes d'affinité, produisant un mélange de peptides et
- détecter au moins un peptide dans le mélange résultant, en utilisant l'analyse de chromatographie liquide-spectrométrie de masse avec détection spectrométrique de masse subséquente, ledit au moins un peptide à détecter étant choisi parmi NHLQG(K) (SEQ ID n° 11), EVATEGI(R) (SEQ ID n° 12) et MVLYTL(R) (SEQ ID n° 13).

2. Procédé selon la revendication 1, dans lequel l'au moins un peptide marqué standard est une NT-proBNP recombinante complète.

3. Procédé selon la revendication 1, dans lequel ledit au moins un peptide standard est choisi parmi
NHLQG(K) (SEQ ID n° 11),
EVATEGI(R) (SEQ ID n° 12) et
MVLYTL(R) (SEQ ID n° 13).

4. Procédé selon la revendication 3, dans lequel ledit au moins un peptide standard est EVATEGI(R) (SEQ ID n° 12).

5. Procédé selon la revendication 3, dans lequel ledit au moins un peptide standard est un EVATEGI(R) (SEQID n° 12) marqué au deutérium.

6. Procédé selon la revendication 1, dans lequel lesdites billes d'affinité sont des billes d'affinité magnétiques.

7. Procédé selon la revendication 1, dans lequel ledit au moins un anticorps se lie spécifiquement à un épitope non glycosylé sur la NT-proBNP avec une séquence d'acides aminés choisie parmi SEQ ID n° 2 et SEQ ID n° 3.

8. Procédé selon la revendication 1, dans lequel la quantification est effectuée sur la base de la calibration contre la NT-proBNP recombinante digérée enzymatiquement.

9. Procédé selon la revendication 1, dans lequel le procédé est calibré en utilisant une NT-proBNP standard trypsinisée à des concentrations pertinentes et tous les standards ayant un pic avec un standard interne.

10. Procédé selon la revendication 9, dans lequel le standard interne est la NT-proBNP complète recombinante.

11. Procédé selon l'une quelconque des revendications 1 à 10 destiné à être utilisé dans l'assignation de valeurs à des matériaux de calibration, le test et/ou le contrôle de qualité d'essais pour déterminer une concentration de pro-hormone BNP N-terminale humaine (NT-proBNP) dans un échantillon.

12. Procédé selon l'une quelconque des revendications 1 à 10 destiné à être utilisé dans le diagnostic et le suivi de l'insuffisance cardiaque.
